(19)

Europäisches
Patentamt
European
Patent Office
Office européen
des brevets

(11) **EP 3 312 748 A1**

(12) **EUROPEAN PATENT APPLICATION**

(43) Date of publication:
25.04.2018 Bulletin 2018/17

(51) Int Cl.:
***G06F 19/00*** (2018.01)

(21) Application number: **17166098.8**

(22) Date of filing: **11.04.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **19.10.2016 DE 102016220530
19.10.2016 GB 201617696**

(71) Applicant: **FUJITSU LIMITED
Kawasaki-shi,
Kanagawa 211-8588 (JP)**

(72) Inventor: **HU, Bo
Winchester, SO23 7DT (GB)**

(74) Representative: **Ward, Gregory Peter
Haseltine Lake LLP
Lincoln House
300 High Holborn
London WC1V 7JH (GB)**

(54) **METHOD FOR AIDING A DIAGNOSIS, PROGRAM AND APPARATUS**

(57) Embodiments of the invention provide a method for aiding a diagnosis for diagnosing a patient with a medical condition, the method comprising: determining a plurality of factors having values that can be used to classify the patient, and a number of levels of abstraction between raw data and a value of a factor; generating a neural network in accordance with the determined number of levels; acquiring raw data in relation to each of the plurality of factors from a plurality of sources; processing the raw data for a factor using the neural network to obtain a value for the factor, classifying the patient using the value for the factor, comparing the classification information for the patient with equivalent information for other subjects, and outputting information for us in diagnosing the patient on the basis of the comparison. Further embodiments of the invention provide a diagnostic aid, and a program for causing a computer to execute the method.

Figure 1

## Description

. The invention relates to a method for aiding a diagnosis, diagnostic program and diagnostic apparatus.

. Existing diagnostic techniques for diagnosing medical conditions in patients often rely heavily on the expertise of medical professionals to provide a correct diagnosis. Often, a patient will present to a medical professional having suffered from one or more symptoms. The medical professional then discusses the symptoms with the patient and orders any diagnostic tests that are believed to be necessary. Diagnostic tests can include using nuclear medicine techniques, analysing the blood or other bodily fluids and tissues of the patient, taking an electrocardiogram (ECG) to monitor the heart of the patient, and so on.

. Once the results of the diagnostic tests are available, the medical professional then typically relies on personal knowledge or experience, potentially in conjunction with consultations with colleagues or medical textbooks, to provide a diagnosis for the patient.

. The diagnostic procedure relies upon the medical professional responsible for providing the diagnosis investing a large amount of time and effort in providing the diagnosis. Unfortunately, there are several drawbacks to this approach. Many areas, both in terms of geographical areas and areas of medical specialisation, suffer from shortages of trained medical professionals. This can mean that the medical professionals required to perform a diagnosis are not available at all, or that medical professionals that are available are not able to devote the required amount of time and effort to each patient due to the burden of their work load. Also, it is not feasible for every medical professional to have knowledge and/or experience of every potential medical condition that a patient may be suffering from. Some conditions are rare or poorly documented in medical textbooks, and medical professionals who are early in their careers may not have the necessary degree of experience to rely upon.

. Accordingly, it is desirable to provide a system to aid in the diagnosis of patients. It is further desirable to if the system is able to relieve some of the time and effort burden on medical professionals, to help address the problems discussed above.

. Prior art systems exist through which a patient profile can be compared with a database of existing profiles in order to assist a medical professional in providing a diagnosis.

. WO 2009/083833 A1 discloses a system wherein a clinical profile of a patient is compared with a database containing a plurality of clinical profiles, resulting in a set of matching clinical profiles. The matching clinical profiles can also be weighted, to indicate which clinical profile from the set of clinical profiles offers the best match to the clinical profile of the patient. The system relies on the input patient data being provided in the same format as the data stored in the database; both must be provided in the clinical profile format. This is not always possible, as a significant amount of work can be required to compile a clinical profile for a patient and all of the necessary information to create a clinical profile may not be available.

. It is desirable to address some of the issues with existing systems.

. According to an aspect of an embodiment of the present invention, there is provided a method for aiding a diagnosis of a patient with a medical condition from among a plurality of medical conditions, the method comprising: determining a plurality of factors, the values of which can be used to classify the patient; determining a number of levels of abstraction between raw data and the value of a factor from among the plurality of factors; generating a neural network in accordance with the number of levels of abstraction between the raw data and the value of the factor; acquiring raw data in relation to each of the plurality of factors, the raw data being acquired from a plurality of sources; processing the raw data using the neural network to derive the value for the factor; classifying the patient using the value derived for the factor, thereby obtaining classification information for the patient; comparing the obtained classification information for the patient with equivalent classification information for one or more subjects; and outputting information for use in diagnosing the patient with the medical condition, on the basis of the comparison. The method provides an efficient means for assisting a medical practitioner in the diagnosis of a patient using data from a plurality of sources.

. A further aspect of an embodiment of the present invention provides a method for aiding a diagnosis wherein the data from the plurality of sources is heterogeneous. By allowing the use of heterogeneous data, the versatility and adaptability of the method to a range of medical conditions is improved.

. A further aspect of an embodiment of the present invention provides a method for aiding a diagnosis wherein each of the sources is assigned a weighting that is representative of the reliability of the source. This reduces the impact of unreliable sources on the overall accuracy of the diagnosis results provided.

. A further aspect of an embodiment of the present invention provides a method for aiding a diagnosis wherein each of the factors is assigned a weighting that is representative of the importance of the factor. This helps to ensure that factors that can be key in diagnosing a disease are given greater prominence in the comparison process.

. A further aspect of an embodiment of the present invention provides a method for aiding a diagnosis wherein the plurality of factors include one or more of social factors, environmental factors, physical factors, family history factors and personal history factors. The inclusion of the various types of factors helps to ensure that information that is key to the diagnosis of a medical condition is taken into consideration during the method for aiding a diagnosis.

. A further aspect of an embodiment of the present invention provides a method for aiding a diagnosis wherein the plurality of sources of raw data include one or more of patient reported symptoms, physician detected symptoms, ECG readings, BMI and blood pressure readings. Use of a variety of sources helps to provide greater diagnostic certainty, and also prevent misdiagnosis due to an anomalous result from a source.

. A further aspect of an embodiment of the present invention provides a method for aiding a diagnosis wherein the medical condition is a mental health condition. The separate analysis of factors to provide characterisation information for a patient are well suited to aiding the diagnosis of mental health problems.

. A further aspect of an embodiment of the present invention provides a method for aiding a diagnosis wherein the classification information for the patient is generated in the form of a multi-dimensional vector. Use of a multi-dimensional vector format improves the speed and ease with which multiple sets of equivalent classification information from a database can be searched.

. A further aspect of an embodiment of the present invention provides a method for aiding a diagnosis wherein the equivalent classification information is in the form of a multi-dimensional vector, and wherein the comparing is performed using cosine similarity. Use of cosine similarity provides an objective and fast comparison between classification information.

. A further aspect of an embodiment of the present invention provides a method for aiding a diagnosis wherein either a different neural network is used to process raw data for each factor, or wherein the neural network is retrained between processing raw data for different factors. Use of plural or retrained neural networks improves the parallelisation of the training process, or reduces the computing requirements of the method.

. A further aspect of an embodiment of the present invention provides a method for aiding a diagnosis wherein at least one neural network is a restricted Boltzmann machine. Restricted Boltzmann machines are particularly well suited to performing the analysis required for the present method in a rapid way.

. A further aspect of an embodiment of the present invention provides a method for aiding a diagnosis wherein the raw data is converted before input into the restricted Boltzmann machine to be in a binary form. Restricted Boltzmann machines are more efficient at operating with data in a binary format rather than, for example, continuous variable data.

. A further aspect of an embodiment of the present invention provides a method for aiding a diagnosis wherein a restricted Boltzmann machine is trained using contrastive divergence algorithms. Contrastive divergence algorithms are a fast and efficient means for training restricted Boltzmann machine type neural networks.

. A further aspect of an embodiment of the present invention provides a method for aiding a diagnosis wherein the number of levels of abstraction between raw data and the value of a factor is determined using ontology, the ontology using information retrieved from online sources and/or locally input information. The use of ontology to help guide the construction of a neural network allows domain knowledge specific higher level feature to be taken into consideration in the training of lower level features.

. A further aspect of an embodiment of the present invention provides a computer program which, when executed by a computing apparatus, causes the computing apparatus to execute the method for aiding a diagnosis. The nature of the calculations required for the method for aiding a diagnosis, both in terms of repetitiveness and complexity, make the method particularly suited to computer implementation.

. According to an aspect of an embodiment of the present invention, there is provided a diagnostic aid, which provides the same benefits as discussed above in the context of the method for aiding a diagnosis.

## Description of Figures

. The invention will now be further described, by way of example only, with reference to the following figures, in which:

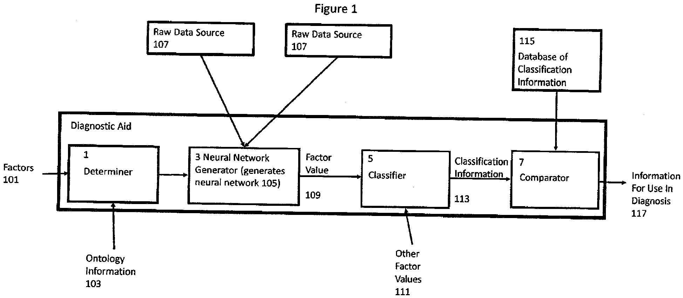

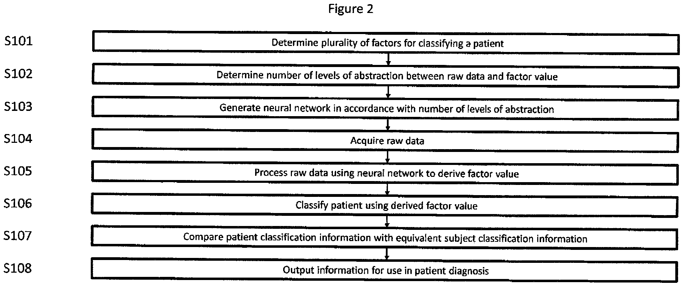

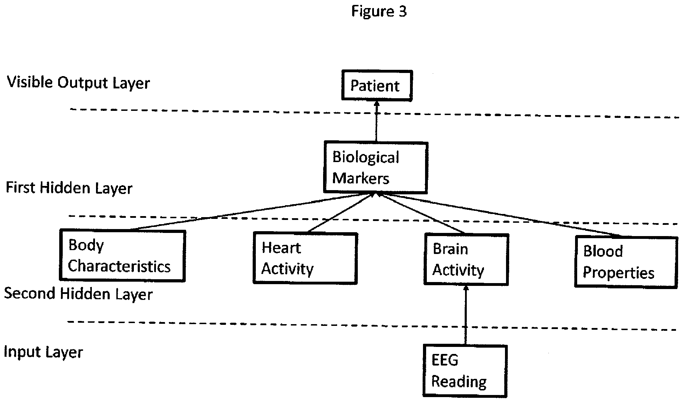

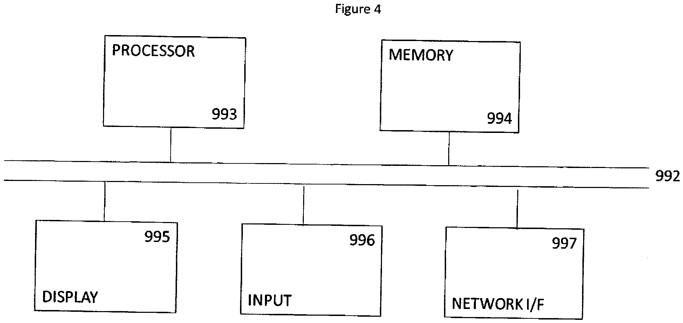

. Figure 1 is a schematic representation of a diagnostic aid according to an aspect of an embodiment of the invention.
. Figure 2 is a flow chart illustrating a method in accordance with an aspect of an embodiment of the invention.
. Figure 3 illustrates a section of an ontology driven model for use as the basis for a neural network according to an aspect of an embodiment of the invention.
. Figure 4 a schematic representation of a computing device that can be used to implement aspects of embodiments of the present invention.

## Detailed Description

. The schematic representation in Figure 1 illustrates a diagnostic aid in accordance with an aspect of an embodiment of the invention. Figure 2 provides a flowchart illustrating a method in accordance with an aspect of an embodiment of the invention. As shown in the schematic of Figure 1, the diagnostic aid includes a determiner 1. The determiner 1 is configured to determine one or more factors 101 that can be used to classify a patient, as shown in step S101 of Figure 2. Typically the factor or factors are input by a medical professional, or are retrieved from a data store (either a local data store or an online data store). The factors 101 are situations or conditions which can help diagnose a medical condition in a patient. The factors 101 may contribute to causing the medical condition; examples of factors which can contribute to a medical condition include social factors (such as whether a patient has a job, whether a patient has friends, whether a patient is in a relationship, etc.), environmental factors (such as whether a patient has a home, whether a patient lives in a polluted area, etc.) and physical factors (whether a patient has generally good physical health). The factors may also be symptoms of a medical condition (such as the presence of certain proteins in blood samples, nausea, etc.)

. Once the determiner 1 has determined a factor 101, the determiner 1 is configured to use the factor 101 as the basis for the planning of a neural network. In particular, a neural network is planned wherein the factor is modelled and it is determined how many levels of abstraction are present between input data (that is, data in the form in which it is input into the neural network) and final values for the factor that can be subsequently used in the classification of a patient, as shown in step S102 of Figure 2.

. In an exemplary embodiment of the modelling, the classification information 113 for each patient is to be provided in the form of a single numerical entity. The embodiment of the invention shown in Figure 1 delivers classification information 113 for each patient in the form of a multi-dimensional vector, wherein each factor 101 that can be used to help classify the patient contributes to the multi-dimensional vector.

. It is preferable, although not essential, if the data to be input into the visible (input) layer of the neural network is provided in a binary form. If the data is not already in a binary form, this may necessitate the conversion of the data. Although the conversion of continuous value data into binary data increases the number of inputs into the neural network, it also significantly reduces the complexity of the input and improves the processing performance of the neural network. An example of the conversion process is provided below, wherein the raw data to be input is the body mass index (BMI) of the patient, which is not provided in a binary form and which therefore requires conversion.

. Body mass index categorises the mass of a patient relative to the height of the patient, in order to define the patient as one of (typically) 4 weight categories: underweight, normal weight, overweight or obese. Although the definitions of the boundaries between the categories vary on a country by country basis, the generally accepted bounds are: BMI < 18.5 = underweight; $18.5 \leq BMI < 25$ = normal weight; $25 \leq BMI < 30$ = overweight; $30 \leq BMI$ = obese. The BMI of a patient is calculated using equation 1, wherein $M$ is the mass of the patient in kilograms (kg) and $h$ is the height of the patient in metres (m).

## Equation 1

$$BMI = \frac{M}{h^2}$$

. Both the mass and height of the patient are continuous variables (rather than discrete variables), so the BMI generated is also a continuous variable. Accordingly, it is preferable to convert the BMI raw data into a binary format for input into the neural network. In an aspect of an embodiment of the invention the conversion involves indicating which of the 4 weight categories the patient falls into, and returning null values for the other categories. As an example of this, a patient with a weight of 70.0 kg and a height of 1.80 m would have a BMI of 21.6 (all to 3 significant figures), so this patient would be considered to fall within the normal weight category. The raw data provided to the diagnostic aid would be the BMI of the patient, 21.6. This can be converted for use in the neural network into a binary form by indicating, in a 4 element vector, the weight category into which the patient falls. For example, the general form of the vector <underweight, normal weight, overweight, obese> would return a specific reading for this patient of <0, 1, 0, 0>.

. Other raw data is preferably converted in an equivalent way, using as many elements as necessary. As an example of this, blood pressure could be entered as a binary vector with a plurality of elements. A blood pressure reading typically comprises two pressure values, one each for systolic and diastolic pressure, both of which are measured by convention in units of mmHg. The conversion factor between mmHg and kPa is 0.133, such that a variation in 1 kPa is equal to approximately a variation of 7.5 mmHg. Typical adult values are in the range of 100/70 mmHg (systolic/diastolic), and the normally measured range is between approximately 70/40 and approximately 190/100.

. The normal recorded range can be represented in a similar fashion to the BMI example discussed above by dividing the systolic into categories of 10 mmHg width, and dividing the diastolic pressure into categories of 5 mmHg width. Further upper and lower categories can also be included to allow values falling outside the normally measured range (that is, below 70 or above 190 systolic, and/or below 40 or above 100 diastolic) to be represented. In this example, the resulting binary vector would have 28 elements, corresponding to: measured systolic pressure < 70, $70 \leq$ measured systolic pressure < 80, $80 \leq$ measured systolic pressure < 90, ..., measured systolic pressure > 190, measured diastolic pressure < 40, $40 \leq$ measured diastolic pressure < 45, ..., measured diastolic pressure > 100. Using this scheme, a common adult reading of 100/70 would be entered as <0, 0, 0, 1, 0, 0, 0, 0, 0, 0, 0, 0, 0, 0, 0, 0, 0, 0, 0, 0, 1, 0, 0, 0, 0, 0, 0, 0>. The categories presented here are one option that is included to illustrate a potential categorisation; other conversions into binary format could also be used.

. If required, the conversion from input raw data is typically performed prior to the first stage of processing by the neural network as a separate process (using a converter, not shown in Figure 1) prior to entering the data into the neural network.

. Once the form of the data to be input into the neural network is known, and given the desired end point of the modelling process, the next step in the modelling is to determine what is required from the neural network to process the input data (either in raw form or converted into a binary form if applicable) and provide the one or more elements to be contributed to the multi-dimensional vector. The processing requirements placed on the neural network to go from the data to the one or more elements determines how many layers of abstraction are required in the neural network, specifically how many hidden layers, and also how many hidden units are provided in each layer.

. The determination of how many hidden layers and units are required is preferably ontology driven; although the determination can also be driven by other factors (such as the computing resources available). The ontology driven approach uses a consideration of interrelationships between various types of input raw data and factors to design the neural network, rather than using a neural network with arbitrary numbers of hidden layers and units.

. The use of ontology to drive the design of the neural network provides a hierarchical structure for the resulting network design. In the resulting network, knowledge of the higher level factors (or sub-factors) can be used to help guide training of the portions of the neural network responsible for lower level factors. Also, compartmentalising the overall generation of the classification information 113 for the patient, whereby the factors generating values that can be used to classify the patient are analysed separately, allows a greater degree of parallelisation of the analysis process.

. Some aspects of embodiments of the invention utilise neural networks to process raw data and derive values for several factors from among the plurality of factors whose values can be used to classify the patient. In certain aspects, neural networks may be used to process raw data for all of the plurality of factors. In cases where several neural networks are used (to analyse raw data for some or all of the factors), training the neural networks can involve a significant time and computing power requirement. By using a different neural network for each factor, the total time required for training can be reduced. Alternatively, by retraining a neural network before processing data for each factor, which essentially amounts to using a different neural network for each factor, the computing power requirements can be reduced.

. Typically, the ontology is determined in accordance with existing standards. As an example, the World Health Organisation (WHO) International Statistical Classification of Diseases and Related Health Problems (ICD) can be used to provide guidance in relation to an ontology that can be used to guide the modelling of the neural network. The ICD provides codes that can be used to track symptoms and diagnoses, and to indicate potential links or related diagnoses. Further potential sources are specific to the types of medical diagnoses sought, for example, the classifications provided in the Diagnostic and Statistical Manual of Mental Disorders, 4th Edition, Text Revision (DSM-IV Codes) relate exclusively to mental health disorders. The exact mechanism for the generation of the ontology is beyond the scope of the present invention, and will typically rely to a certain degree on the expertise of one or more medical professionals. In circumstances where there is no existing ontology that can be used to drive the design of the neural network, a knowledge model is created with the involvement of experts in the relevant medical domain. Such experts will be well aware of the established methodologies for knowledge acquisition and knowledge representation. Generation of a suitable ontology typically involves input from programming specialists and knowledge engineers, in addition to experts in the medical domain. In general, an ontology is represented in a computer understandable coding system. Once a suitable ontology has been developed or identified, information on these ontologies can be stored online for retrieval as required. Systems for hosting ontologies (and related information) online already exist, for example the LOD4All.net repository which is used to store ontologies for use or adaptation.

. The ontology driven approach is explained below with reference to Figure 3. Figure 3 shows an example of a section of an ontology driven model used as the basis of a neural network. The factor that can be used to classify the patient in this instance is "Biological Markers", that is, physical readings taken from the patient's body which may be indicative of a particular medical condition. Accordingly, there is a hidden unit in the first hidden layer of the neural network (that is, the layer below the visible output layer, which is the Patient layer) that is associated with Biological Markers. Sub-factors which influence the values of the Biological Markers factor include: Body Characteristics (such as BMI), Heart Activity, Blood Properties (such as blood pressure) and Brain Activity. These sub-factors are illustrated in Figure 3. These sub-factors are each associated with a hidden unit in the next hidden layer of the neural network; the second hidden layer that is below the first hidden layer, as shown in Figure 3. The outputs from the second hidden layer form the inputs for the first hidden layer.

. The Brain Activity sub-factor is influenced by the input data in the next level shown in Figure 3, an example of which is indicated by the electroencephalography (EEG) section. Electroencephalography involves monitoring electrical activity in the brain, generally using a plurality of electrodes attached to the scalp of a patient although electrodes implanted directly in the brain are also used in some circumstances. The electroencephalograph (EEG reading) generated can be used to help diagnose certain medical conditions, such as epilepsy.

. The EEG reading is interpreted by searching the reading for waveforms of different frequencies, and determining whether these waveforms are present or not and, if present, if the waveforms are normal or abnormal. The presence or absence of normal or abnormal waveforms can be indicative of certain medical conditions. Examples of the waveforms which may be detected are provided in Table 1 below, along with typical frequencies of the waveforms in Hertz (Hz).

**Table 1**

| Waveform | Frequencies (Hz) |
|---|---|
| Delta ($\delta$) | <4 |
| Theta ($\theta$) | 4-7 |
| Alpha ($\alpha$) | 8-15 |
| Beta ($\beta$) | 16-31 |
| Gamma ($\gamma$) | >32 |
| Mu ($\mu$) | 8-12 |

. The portion of the diagnostic aid handling the EEG reading can be configured to receive the EEG reading (in a suitable file format) and interpret the EEG reading. That is, this portion of the diagnostic aid can be trained to detect if the waveforms listed above are present and, if present, if they are normal or abnormal. Alternatively, a medical practitioner can analyse the EEG and input the present/absent and normal/abnormal information. In the event the diagnostic aid performs the EEG analysis and the (preferred) binary output is generated, an example resultant vector output (to be used as an input for the Brain Activity unit in the hidden level above) would have 12 variables and be of the form <$\delta$ present, $\delta$ abnormal, $\theta$ present, $\theta$ abnormal, ... $\mu$ abnormal>. Therefore, a vector for an EEG reading that was analysed as containing $\delta$, $\alpha$ and $\beta$ waves of which the $\delta$ wave was abnormal, would be <1, 1, 0, 0, 1, 0, 1, 0, 0, 0, 0, 0>. In the aspect of an embodiment of the invention shown in Figure 3, this vector is passed to the Brain Activity unit of the neural network. This unit outputs to the Biological Markers unit which, in turn, outputs factor values 109 to be used in the classifier 5.

. In an aspect of an embodiment of the invention, the neural network used to process the raw data to derive a value for the factor is a restricted Boltzmann machine (RBM). Restricted Boltzmann machines are a class of stochastic neural networks (having random variations introduced into the network) that are particularly suited to applications involving classification. In RBMs, there are no connections between units within a layer of the neural network; essentially the units within two adjacent layers (such as a visible layer and a hidden layer, or two adjacent hidden layers) form a bipartite graph wherein all the units in one of the layers form a part of the bipartite graph. More specifically, in aspects of embodiments wherein every unit in a layer is connected to every unit in an adjacent layer (but connected to no units in the same layer), the units form a fully connected bipartite graph. In a RBM, a bias is associated with each unit in the layers.

. RBMs are energy based models, wherein an energy function is used to compute probabilities in the model. The energy function is defined using equation 2 as shown below, wherein there are $n$ visible units in the visible layer and $m$ hidden units in the hidden layer, $w_{j,i}$ is the weight assigned to the edge connecting hidden unit $h_j$ and visible unit $v_i$, $a_i$ is the bias associated with visible unit $v_i$, $b_j$ is the bias associated with hidden unit $h_j$ $\mathbf{v}$ the vector of visible units and $\mathbf{h}$ the vector of hidden units.

## Equation 2

$$E(\mathbf{v}, \mathbf{h}) = -\sum_{i=1}^{n} a_i v_i - \sum_{j=1}^{m} b_j h_j - \sum_{i=1}^{n} \sum_{j=1}^{m} h_j w_{j,i} v_i$$

. The energy function $E(\mathbf{v}, \mathbf{h})$ can then be used to compute probabilities, as the probability distributions over the visible and hidden vectors are defined in terms of the energy function. This is shown in equation 3, where $\mathbf{Z}$ is a normalising constant that ensures the probability distribution sums to 1.

## Equation 3

$$P(v, h) = \frac{1}{Z} \sum_h e^{-E(v,h)}$$

. Accordingly, the probability of *v* given *h* (that is, $P(v|h)$) and of *h* given *v* are given by the equations shown in equation 4.

## Equation 4

$$P(v|h) = \prod_{i=1}^{m} P(v_i|h) \quad \text{and} \quad P(h|v) = \prod_{j=1}^{n} P(h_j|v)$$

. Using the energy function, the values of $P(h_j = 1|\mathbf{v})$ and $P(v_i = 1|\mathbf{h})$ can therefore be calculated.

. Use of RBM neural networks is advantageous for some aspects of embodiments of the present invention for several reasons. A large number of factors can be required to identify a specific medical condition, meaning that raw data may be input in a wide variety of formats, which can be handled using a series of linked RBMs. Further, the training of the neural networks can be performed more quickly and efficiently if the neural networks are RBMs, because certain narrowly applicable training algorithms can be applied to RBMs that are not suitable for other neural networks. An example of a training algorithm which is used in aspects of embodiments of the present invention wherein the neural network is a RBM is a contrastive divergence algorithm.

. Contrastive divergence algorithms are used to estimate the gradient of an energy function in an efficient manner, without requiring the value of the energy function itself to be evaluated. Contrastive divergence is used to train the weights and biases in an RBM. With reference to equation 4, the hidden layer can be updated by sampling visible layer using the above probability. Given a randomly selected training set $\mathbf{v}^k$, the probability of a hidden unit is set to 1 is given by $P(h_j = 1|\mathbf{v})$; given a hidden vector of $\mathbf{h}^l$, the state of the visible units are estimated as $P(v_i = 1|\mathbf{h})$. The learning rule is given by equation 5, where subscript *d* is based on data distribution and *r* is based on the reconstructed or updated data.

## Equation 5

$$\Delta w_{ij} = \mathbb{E}\left(v_i, h_j\right)_d - \mathbb{E}\left(v_i, h_j\right)_r$$

. Once the number of levels of abstraction between raw data and the value of a factor from among the plurality of factors has been determined, this information is then passed on to the neural network generator 3, as shown in Figure 3. The neural network generator 3 is responsible for generating and training the neural network 105, as shown in step S103 of Figure 2. The neural network is generated in accordance with the neural network structure determined by the determiner 1. The generated neural network is then trained as required.

. Once the neural network has been generated and trained, the raw data in relation to the given factor from among the plurality of factors is input into the neural network, after conversion if applicable. The acquisition of the raw data is shown is shown in step S104 of Figure 2. The raw data is provided from a plurality of raw data sources 107, as shown in Figure 1. Although it is possible for all the raw data in relation to a particular factor to be provide in the same format (because all of the raw data sources 107 providing raw data in relation to that factor use the same format), it is more common for heterogeneous, multimodal raw data to be input into the diagnostic aid. Considering the example discussed above (and shown in Figure 3) wherein the factor for which the neural network is used to calculate values is Biological Markers, multimodal sources 107 of raw data for this factor could include EEG readings, blood pressure results, x-ray or magnetic resonance imaging (MRI) images, and so on. Because the diagnostic aid is capable of accepting heterogeneous data from the plurality of (different) sources 107, the diagnosis aid is more able to adapt to the available information and provide a diagnosis. Once acquired, the raw data is processed using the neural network, as shown in step S105 of Figure 2.

. The processing of the input data by the neural network results in the generation of a factor value 109. As discussed above, the factor value 109 is preferably provided in the form of a multi-dimensional vector, however other formats such as tables of results can also be used.

. The factor value 109 is then passed to the classifier 5, as shown in Figure 1. The classifier 5 is responsible for classifying the patient on the basis of the factor value 109 obtained from the neural network (as discussed above) along with additional factor values 111, as shown in step S106 of Figure 2. The additional factor values 111 may also be obtained using neural networks (either other neural networks or the same neural network that has been retrained, as discussed above), or may alternatively be obtained in a different way.

. Using the collated factor values for the patient, the classifier 5 defines the patient using classification information 113. In an aspect of an embodiment of the invention, the classification information 113 is provided in the form of a multi-dimensional vector, however other formats can also be used to present the classification information 113. For example, the classification information 113 can be provided in the form of a table which lists the values for various factors. Alternatively, the information can be provided in the form of a written summary, or a graphical representation of the different factors.

. The classification information 113 is then compared to equivalent classification information that is obtained from a database of classification information 115, as shown in step S107 of Figure 2. The database 115 contains stored classification information that is in the same format as the patient classification information 113 that is generated by the classifier 5 (using the obtained factor values 109 for the patient). Each of the sets of stored classification information is linked to a patient case file, which contains relevant information for providing a diagnosis. Examples of stored information can include information on the patient (age, gender, etc.) and the medical professionals responsible for treating the patient, what symptoms the patient presented, what diagnosis was arrived at by the medical professionals, what treatment options were considered or tried, the prognosis for the patient, links to other relevant case files or medical information, and so on.

. Although it is possible for the database 115 to be stored locally, for example, on a storage unit of a desktop computer or a server connected to a network of a single hospital, it is preferable for the database 115 to be stored online. This is because use of an online database 115 allows access to a far larger amount of stored patient information than is possible using an isolated localised system, and also allows the stored patient information to be both accessed and uploaded on a national or global level. If necessary, it is possible for a medical professional to limit the stored classification information that can be compared to the classification information 113 for the patient to be diagnosed. That is, a medical professional can specify that only stored classification information linked to patient having certain qualities should be considered for comparison. This can be useful for increasing the speed of a search, for example, when a medical professional believes that the medical condition from which a patient is suffering is only found in a subset within the overall group of patient cases. An example of this would be a situation wherein the patient is suffering from a condition linked to sexual characteristics, in which case stored classification information linked to cases of patients of a different gender to the patient to be diagnosed may be considered by a medical professional unlikely to provide useful information and may accordingly be excluded from the comparison.

. The diagnosis of medical conditions often requires performing a comparison between a case (that is, the symptoms suffered by a given patient) and existing diagnosed cases from literature (such as medical case reference guides or medical journals). It can be difficult to perform this comparison without a significant amount of work by medical professionals and, even with the work of medical professionals, it can be difficult to perform the comparison in an objective way or to search a large number of reference cases for the best match. Objectivity can be compromised, for example, because it is common for medical professionals when considering medical information from a remote area of medicine outside their specific medical area of expertise to rely on an analysis of the medical information from the remote area of medicine that has been provided by a further medical professional that is active in the remote area of medicine, rather than considering the medical information itself.

. As an example of the above discussed situation, a general practitioner wishing to use information from an x-ray image may be tempted to rely on a written interpretation of the x-ray provided by a radiographer, rather than consulting the x-ray image itself. If the further medical professional (the radiographer in this example) originally obtained and considered the medical information (the x-ray image) with a specific condition in mind, it is possible that the further medical professional may have overlooked or failed to report on symptoms evident in the medical information but unrelated to the specific condition. This situation could result in the original medical professional (the general practitioner in this example) remaining unaware of the symptoms, because the symptoms are present in the original medical data but absent from the analysis.

. The problem discussed above can be addressed by aspects of embodiments of the invention, because the raw data is fed into the diagnostic aid for analysis, so the subjective or focussed interpretation and analysis provided by the further medical practitioner (the radiographer in the above example) is not used as input data to the system.

. In an aspect of an embodiment of the present invention, the provision of the equivalent classification information in the same format as the classification information 113 for the patient to be diagnosed makes the comparison easier to perform. In particular, it is faster and easier to compare two sets of classification information either using technological means (such as image analysis in the case of a graphical representation of classification information), or by a visual comparison performed by a medical professional. Aspects of embodiments of the present invention allow the comparison to be performed by a comparator 7, as shown in Figure 1. Although the comparator 7 in some aspects of embodiments of the invention can be configured to perform the comparison using image analysis, it is preferable, and more efficient, if the classification information is provided in the form of a multi-dimensional vector, as discussed above

. When the classification information 113 of the patient and the classification information stored in the database 115 are provided in the form of multi-dimensional vectors, the comparison can be performed by taking the cosine similarity of the two vectors. That is, the classification information 113 for the patient can be compared with a classification information for series of stored patients by taking the cosine similarity between the stored patient multi-dimensional vector and the patient multi-dimensional vector.

. The cosine similarity is a measure of similarity between two vectors, and is calculated using equation 6 wherein $A$ and $B$ are the multi-dimensional vectors obtained from the classifier 5 and the database 115 respectively, each having $n$ components. The symbols $A_i$ and $B_i$ indicate the $i$th components of $A$ and $B$ respectively.

## Equation 6

$$cos\theta = \frac{A \cdot B}{|A||B|} = \frac{\sum_{i=1}^{n} A_i B_i}{\sqrt{\sum_{i=1}^{n} A_i^2}\sqrt{\sum_{i=1}^{n} B_i^2}}$$

. The output from equation 6 is the cosine similarity value, which is a value between 0 and 1 that indicates the similarity between the two input vectors, wherein a value of 1 indicates that the comparison was made between two sets of identical classification information and lower values indicate that the comparison was between more dissimilar sets of classification information. The use of an entirely numerical comparison metric provides a more objective means for comparing classification information than mechanisms that are based on, or require elements of, medical professional judgement. Also, because the calculations required are comparatively simple, aspects of embodiments of the present invention that use cosine similarity to compare the classification information 113 provided by the classifier 5 for a patient with the stored classification information from the database 115 can quickly compare a large number of stored classification information sets with the patient classification information 113, increasing the chances that a close match will be detected.

. Once the comparison has been performed by the comparator 7, the comparator 7 is configured to output information for use in a potential diagnosis 117 for the patient on the basis of the comparison results, so that the information can be considered and a diagnosis provided by a medical professional. This is shown in step S108 of Figure 2. The comparator 7 can be configured to output a single patient case file, for example, the patient case file that is linked to the stored classification information that was determined by the comparator 7 to be the most similar to the classification information 113 for the patient as obtained by the classifier 5. However, the comparator 7 can alternatively be configured to provide a plurality of patient case files selected from among the patient case files linked to stored classification information that was found to be amongst the most similar to the patient classification information 113 obtained by the classifier 5. This can be of particular use in cases where an exact match for the patient classification information 113 is not located amongst the stored classification information from the database 115, as the provision of a series of case files can allow a medical professional to make an informed judgement as to which of the patient case files linked to the stored classification information appears most relevant to the patient to be diagnosed, or should be investigated further.

. In aspects of embodiments of the invention wherein the comparator 7 is configured to provide a plurality of patient case files (linked to a plurality of stored classification information sets from the database 115), the comparator 7 can be configured to ensure that the patient case files presented include patients that were diagnosed with a range of different diagnoses (rather than presenting a plurality of patient cases relating to patients who were all diagnosed with the same condition). In this way, it is possible to ensure that a range of options are presented to a medical professional for consideration when making a diagnosis.

. In some aspects of embodiments of the invention, the comparator 7 is further configured to provide statistical information on the comparison process and results in addition to providing the patient cases. This statistical information can include, at a basic level, the similarity measure between the classification information 113 for the patient and the stored classification information linked to the patient cases provided by the comparator 7. Additional information can also be provided, such as the total amount of stored classification information considered, the fraction of the stored classification information providing a match of at least a given quality, the time required for the search, and so on.

. In some aspects of embodiments of the invention, the comparator 7 can be configured to only provide patient cases linked to stored classification information that satisfies a minimum similarity threshold in comparison with the classification information 113 for the patient to be diagnosed. This threshold can be fixed in the diagnostic aid, or alternatively can be adjusted by a medical professional using the diagnostic aid. In the event that a minimum similarity threshold is used and none of the stored classification information in the database 115 linked to the comparator 7 satisfies this threshold when compared to the classification information 113 for the patient to be diagnosed, the comparator 7 can return a message indicating that no similar patient case files have been located in the database 115. The diagnostic aid may be further configured to suggest alternative databases 115 that may be searched.

. After the comparator 7 has identified the patient case files as discussed above, these patient case files are presented to the medical professional that is using the diagnostic aid. Typically, summaries of the patient case files are presented to the medical professional, such that the medical professional can select patient case files to consider in detail. Using a selected patient case file or files, the medical professional (typically a clinician) can then diagnose the patient to be diagnosed with a medical condition, or select further testing to assist in providing a diagnosis. Although the comparator 7 can output information for use in providing a suggested diagnosis based on the comparison, the actual diagnosis should always be provided by a medical professional (such as a clinician) before any treatment is begun.

. Although aspects of embodiments of the present invention are suitable for assisting in the diagnosis of medical problems from any field of medicine, they are particularly suited to assisting in the diagnosis of mental health problems. This is due to the inherent nature of mental health problems.

. Most physical health problems generate a limited set of symptoms and biomarkers which, while not providing a definite indication that the physical health problem is present, strongly indicate that this is the case. For example diabetes causes symptoms including frequent urination, thirst, fatigue, blurred vision and reduced healing ability. Diabetes also causes biomarkers such as increased insulin levels. Accordingly, suspected diabetes is generally easy to detect, and the disease is generally easy to confirm if suspected. Further, because diabetes is a common and generally well understood disease, many medical practitioners are able to diagnose the disease without requiring the use of diagnostic aids.

. By contrast, many mental health problems have symptoms which are less definite and less easy to detect. For example, a medical practitioner may not detect if a patient is suffering from mild depression, or if a patient is having suicidal thoughts. Also, many mental problems cannot be easily confirmed by the detection of one or more biomarkers (such as the levels of certain proteins in a blood sample), because biomarkers may not exist for the mental problem. Further, mental problems can be triggered by a wide range and combination of factors (such as social, environmental and physical factors), requiring a wide range of information sourced from different areas to be taken into consideration. Accordingly, the configuration of aspects of embodiments of the present invention whereby disparate factors can be analysed separately to provide characterisation information for a patient are well suited to aiding the diagnosis of mental health problems.

. Figure 4 is a block diagram of a computing device, such as a personal computer, which embodies the present invention, and which may be used to implement an embodiment of the method for aiding a diagnosis for diagnosing a patient with a medical condition from among a plurality of medical conditions. The computing device comprises a processor 993, and memory 994. Optionally, the computing device also includes a network interface 997 for communication with other computing devices, for example with other computing devices of invention embodiments, or for computing with remote databases.

. For example, an aspect of an embodiment of the invention may be composed of a network of such computing devices, such that components of the diagnostic aid are split across a plurality of computing devices. In particular, where a neural network is utilised, this neural network may be implemented by a plurality of interconnected computing devices. Optionally, the computing device also includes one or more input mechanisms such as keyboard and mouse or touchscreen interface 996, and a display unit such as one or more monitors 995. The components are connectable to one another via a bus 992.

. The memory 994 may include a computer readable medium, which term may refer to a single medium or multiple media (e.g., a centralized or distributed database and/or associated caches and servers) configured to carry computer-executable instructions or have data structures stored thereon. Computer-executable instructions may include, for example, instructions and data accessible by and causing a general purpose computer, special purpose computer, or special purpose processing device (e.g., one or more processors) to perform one or more functions or operations. Thus, the term "computer-readable storage medium" may also include any medium that is capable of storing, encoding or carrying a set of instructions for execution by the machine and that cause the machine to perform any one or more of the methods of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

. The processor 993 is configured to control the computing device and execute processing operations, for example executing code stored in the memory to implement the various different functions of the determiner 1, neural network generator 3, diagnostic aid, classifier 5 and comparator 7 described here and in the claims. The memory 994 stores data being read and written by the processor 993. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and steps discussed herein.

. The display unit 997 may display a representation of data stored by the computing device and may also display a cursor and dialog boxes and screens enabling interaction between a user and the programs and data stored on the computing device. The display unit may also comprise a touchscreen interface. The input mechanisms 996 may enable a user to input data and instructions to the computing device. In particular, the display unit may be used to display the obtained diagnosis or diagnoses, and the input mechanisms may be used to control the view of the obtained diagnosis or diagnoses or to configure the diagnosis aid.

. The network interface (network I/F) 997 may be connected to a network, such as the Internet, and is connectable to other such computing devices via the network. The network I/F 997 may control data input/output from/to other apparatus via the network.

. Other peripheral devices such as microphone, speakers, printer, power supply unit, fan, case, scanner, trackerball etc. may be included in the computing device.

. The determiner 1 of Figure 1 may be a processor 993 (or plurality thereof) executing processing instructions (a program) stored on a memory 994 and exchanging data via a network I/F 997 or bus 992. In particular, the processor 993 can execute processing instructions to determine a plurality of factors, the values of which can be used to classify a patient, as discussed above. Furthermore, the processor 993 may execute processing instructions to store the factors on a connected storage unit and/or to transmit, via the network I/F 997 or bus 992, the factors to the neural network generator 3 for processing if the neural network generator 3 is located remotely from the determiner 1.

. The neural network generator 3 of Figure 1 may be a processor 993 (or plurality thereof) executing processing instructions (a program) stored on a memory 994 and exchanging data via a network I/F 997 or bus 992. In particular, the processor 993 can execute processing instructions to determine a number of levels of abstraction between raw data and the value of a factor from among the plurality of factors, and to generate a neural network in accordance with the determined number of levels, as discussed above. Furthermore, the processor 993 may execute processing instructions to store generated neural network on a connected storage unit and/or to transmit, via the network I/F 997 or bus 992, the instructions for construction of the neural network to a further computing device.

. The classifier 5 of Figure 1 may be a processor 993 (or plurality thereof) executing processing instructions (a program) stored on a memory 994 and exchanging data via a network I/F 997 or bus 992. In particular, the processor 993 can execute processing instructions to classify the patient using the value derived for the factor, thereby obtaining classification information 113 for the patient, as discussed above. Furthermore, the processor 993 may execute processing instructions to store the classification information 113 on a connected storage unit and/or to transmit, via the network I/F 997 or bus 992, the classification information 113 to the comparator 7 for processing if the comparator 7 is located remotely from the classifier 5.

. The comparator 7 of Figure 1 may be a processor 993 (or plurality thereof) executing processing instructions (a program) stored on a memory 994 and exchanging data via a network I/F 997 or bus 992. In particular, the processor 993 can execute processing instructions to compare the obtained classification information 113 for the patient with equivalent classification information for one or more subjects retrieved from a database, and to provide a diagnosis of a medical condition for the patient on the basis of the comparison, the values of which can be used to classify a patient, as discussed above. Furthermore, the processor 993 may execute processing instructions to store the diagnosis or comparison results on a connected storage unit and/or to transmit, via the network I/F 997 or bus 992, the diagnosis or comparison results to a local or remote memory 994 or display 995 if the comparator 7 is located remotely from the memory or display.

. Methods embodying the present invention, for example the method as illustrated by the flowchart of Figure 2, may be carried out on a computing device such as that illustrated in Figure 4. Such a computing device need not have every component illustrated in Figure 4, and may be composed of a subset of those components. A method embodying the present invention may be carried out by a single computing device in communication with one or more data storage servers via a network, as discussed above.

. As the present invention is configured to provide diagnosis suggestions without human intervention in the analysis of patient information, the results of the classification are less subjective than those produced using other methods.

. For the avoidance of doubt, the scope of the invention is defined by the claims.

**Claims**

1. A method for aiding a diagnosis of a patient with a medical condition from among a plurality of medical conditions, the method comprising:

determining a plurality of factors, the values of which to be used to classify the patient;

determining a number of levels of abstraction between raw data and the value of a factor from among the plurality of factors;

generating a neural network in accordance with the number of levels of abstraction between the raw data and the value of the factor;

acquiring raw data in relation to each of the plurality of factors, the raw data being acquired from a plurality of sources;

processing the raw data using the neural network to derive the value for the factor;

classifying the patient using the value derived for the factor, thereby obtaining classification information for the patient;

comparing the obtained classification information for the patient with equivalent classification information for one or more subjects; and

outputting information for use in diagnosing the patient with the medical condition, on the basis of the comparison.

2. The method for aiding a diagnosis according to claim 1, wherein the raw data acquired from the plurality of sources is heterogeneous.

3. The method for aiding a diagnosis according to any preceding claim, wherein each of the sources is assigned a weighting that is representative of the reliability of the source.

4. The method for aiding a diagnosis according to any preceding claim, wherein each of the factors is assigned a weighting that is representative of the importance of the factor.

5. The method for aiding a diagnosis according to any preceding claim, wherein the plurality of factors include one or more of social factors, environmental factors, physical factors, family history factors and personal history factors.

6. The method for aiding a diagnosis according to any preceding claim, wherein the plurality of sources of raw data include one or more of patient reported symptoms, physician detected symptoms, ECG readings, BMI and blood pressure readings.

7. The method for aiding a diagnosis according to any preceding claim, wherein the medical condition is a mental health type condition.

8. The method for aiding a diagnosis according to any preceding claim, wherein the classification information for the patient is generated in the form of a multi-dimensional vector.

9. The method for aiding a diagnosis according to claim 8, wherein the equivalent classification information is in the form of a multi-dimensional vector, and wherein the comparing is performed using cosine similarity.

10. The method for aiding a diagnosis according to any preceding claim, wherein either a different neural network is used to process raw data for each factor, or wherein the neural network is retrained between processing raw data for different factors.

11. The method for aiding a diagnosis according to claim 10, wherein at least one neural network is a restricted Boltzmann machine, optionally wherein the raw data is converted before input into the restricted Boltzmann machine to be in a binary form.

12. The method for aiding a diagnosis according to claim 11, wherein the restricted Boltzmann machine is trained using contrastive divergence algorithms.

13. The method for aiding a diagnosis according to any of claims 10 to 12, wherein the number of levels of abstraction between raw data and the value of a factor is determined using ontology, the ontology using information retrieved from online sources and/or locally input information.

14. A computer program which, when executed by a computing apparatus, causes the computing apparatus to execute the method for aiding a diagnosis of any of claims 1 to 13.

15. A diagnostic aid configured to aid in the diagnosis of patient with a medical condition, from among a plurality of medical conditions, the diagnostic aid comprising:

a determiner configured to determine a plurality of factors, the values of which can be used to classify the patient; and

a neural network generator configured to determine a number of levels of abstraction between raw data and the value of a factor from among the plurality of factors, and to generate a neural network in accordance with the determined number of levels;

the diagnostic aid being further configured to acquire raw data from a plurality of sources and process the raw data using the neural network to derive a value for the factor, and the diagnostic aid further comprising:

a classifier configured to classify the patient using the value derived for the factor, thereby obtaining classification information for the patient; and

a comparator configured to compare the obtained classification information for the patient with equivalent classification information for one or more subjects retrieved from a database, and to output information for use in determining a diagnosis of a medical condition for the patient, on the basis of the comparison.

Figure 1

Raw Data Source
107

Raw Data Source
107

115
Database of
Classification
Information

Diagnostic Aid

Factors
101

1
Determiner

3 Neural Network
Generator (generates
neural network 105)

Factor
Value

109

5
Classifier

Classification
Information

7
Comparator

Information
For Use In
Diagnosis
117

113

Ontology
Information
103

Other
Factor
Values
111

Figure 2

| S101 | Determine plurality of factors for classifying a patient |
|---|---|
| S102 | Determine number of levels of abstraction between raw data and factor value |
| S103 | Generate neural network in accordance with number of levels of abstraction |
| S104 | Acquire raw data |
| S105 | Process raw data using neural network to derive factor value |
| S106 | Classify patient using derived factor value |
| S107 | Compare patient classification information with equivalent subject classification information |
| S108 | Output information for use in patient diagnosis |

Figure 3

Visible Output Layer

Patient

First Hidden Layer

Biological Markers

Body Characteristics

Heart Activity

Brain Activity

Blood Properties

Second Hidden Layer

Input Layer

EEG Reading

Figure 4

| PROCESSOR |  | MEMORY |
|---|---|---|
| 993 |  | 994 |

992

| 995 | 996 | 997 |
|---|---|---|
| DISPLAY | INPUT | NETWORK I/F |

Europäisches
Patentamt

European
Patent Office

Office européen
des brevets

# EUROPEAN SEARCH REPORT

Application Number

EP 17 16 6098

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | YE ET AL: "Cosine similarity measures for intuitionistic fuzzy sets and their applications", MATHEMATICAL AND COMPUTER MODELLING, PERGAMON PRESS, OXFORD, GB, vol. 53, no. 1-2, 1 January 2011 (2011-01-01), pages 91-97, XP027435994, ISSN: 0895-7177, DOI: 10.1016/J.MCM.2010.07.022 [retrieved on 2010-08-03] * Formula 2; sentences Page 2, last 2 lines; example 2 * | 1-15 | INV. G06F19/00 |
| Y | G. E. HINTON ET AL: "Reducing the Dimensionality of Data with Neural Networks", SCIENCE, vol. 313, no. 5786, 28 July 2006 (2006-07-28), pages 504-507, XP055313000, ISSN: 0036-8075, DOI: 10.1126/science.1127647 * Page 2 column 3 paragraph 2 Page 3 column 1 page 4 column 2 paragraphs 2-5; figure 1 * | 1-15 | |
| A | WO 98/10697 A1 (NEURALMED INC [US]) 19 March 1998 (1998-03-19) * the whole document * | 1-15 | **TECHNICAL FIELDS SEARCHED (IPC)** G06F |
| A | EP 0 842 475 A1 (HORUS THERAPEUTICS INC [US]) 20 May 1998 (1998-05-20) * the whole document * | 1-15 | |

The present search report has been drawn up for all claims

| Place of search | Date of completion of the search | Examiner |
|---|---|---|
| Munich | 5 September 2017 | Rivera, Pedro V. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 3 312 748 A1

## ANNEX TO THE EUROPEAN SEARCH REPORT
## ON EUROPEAN PATENT APPLICATION NO.

EP 17 16 6098

This annex lists the patent family members relating to the patent documents cited in the above-mentioned European search report.
The members are as contained in the European Patent Office EDP file on
The European Patent Office is in no way liable for these particulars which are merely given for the purpose of information.

05-09-2017

| Patent document cited in search report | | Publication date | Patent family member(s) | | Publication date |
|---|---|---|---|---|---|
| WO 9810697 | A1 | 19-03-1998 | AU | 4263997 A | 02-04-1998 |
| | | | US | 5839438 A | 24-11-1998 |
| | | | WO | 9810697 A1 | 19-03-1998 |
| EP 0842475 | A1 | 20-05-1998 | AT | 197511 T | 11-11-2000 |
| | | | AU | 717449 B2 | 23-03-2000 |
| | | | CA | 2227543 A1 | 13-02-1997 |
| | | | CN | 1194045 A | 23-09-1998 |
| | | | DE | 69610926 D1 | 14-12-2000 |
| | | | DE | 69610926 T2 | 21-06-2001 |
| | | | DK | 0842475 T3 | 27-11-2000 |
| | | | EP | 0842475 A1 | 20-05-1998 |
| | | | ES | 2152548 T3 | 01-02-2001 |
| | | | GR | 3035370 T3 | 31-05-2001 |
| | | | JP | H11504739 A | 27-04-1999 |
| | | | JP | 2007052774 A | 01-03-2007 |
| | | | NZ | 315428 A | 28-02-2000 |
| | | | PT | 842475 E | 30-04-2001 |
| | | | WO | 9705553 A1 | 13-02-1997 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009083833 A1